# FASCICULE DE BREVET EUROPEEN

(11) **EP 0 666 255 B1**
(45) Date de publication et mention de la délivrance du brevet: **22.10.1997**
(21) Numéro de dépôt: 95400225.9
(22) Date de dépôt: 02.02.1995
(51) Int. Cl.: C07C 403/10, C07C 403/14

(54) **Procédé de préparation de la vitamine A et nouveaux intermédiaires**
Verfahren zur Herstellung von Vitamin A und Zwischenprodukte
Process for the preparation of vitamin A and intermediates

(30) Priorité: 04.02.1994 FR 9401260
(43) Date de publication de la demande: 09.08.1995
(73) Titulaire: RHONE-POULENC NUTRITION ANIMALE, 92160 Antony (FR)
(72) Inventeur: Ancel, Jean-Erick, F-69008 Lyon (FR); Bienayme, Hugues, F-69002 Lyon (FR)
(74) Mandataire: Le Pennec, Magali

(56) Documents cités:
- EP-A- 0 237 438
- FR-A- 2 434 135

## Description

La présente invention concerne un nouveau procédé de préparation de la vitamine A et de nouveaux intermédiaires obtenus au cours des différentes étapes de ce procédé. Elle concerne plus particulièrement un procédé de préparation de la vitamine A par condensation de l'éthynyl bétaionol et du motif C₅ porteur d'un chlorure allylique.

Il est connu de préparer la vitamine A par un procédé voisin tel que décrit dans le brevet US 4 035 425. Ce brevet décrit la condensation de l'éthynyl bétaionol avec un dérivé chloré allylique porteur d'une fonction ester. La condensation est réalisée en présence de cuivre et d'une base. Le dérivé obtenu lors de cette condensation est un dérivé acétylénique porteur d'un motif hydroxyle ou alkoxyl en a du groupe acétylénique et d'un groupe ester souvent acétate en position 15, de formule suivante :

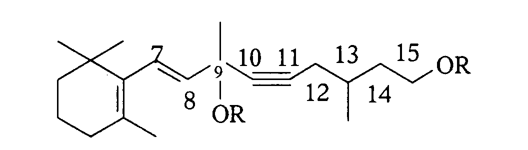

L'hydrogénation partielle du motif acétylénique permet d'obtenir le dérivé éthylénique correspondant qui, après élimination du motif hydroxyle ou du groupe alkoxy situé en position 9 avec l'hydrogène en position 12 (élimination dite 1,4), permet la formation d'une double liaison qui se conjugue avec la double liaison existante, ce qui permet d'obtenir la double liaison 11-12 uniquement sous la configuration trans. Dans ce brevet, il existe une incertitude sur la nature de la liaison 9-10. Les autres liaisons, c'est-à-dire les liaisons 7-8 et 13-14 ne sont pas impliquées dans cette conjugaison, ainsi la vitamine A obtenue dans le brevet US 4 035 425 ne peut être obtenue sous la forme trans que si l'éthynyl β ionol est trans en position 7-8 et si le chloro ester possède aussi une liaison tout trans.

Le chloro ester en C₅ de formule suivante : 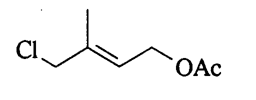 de stéréochimie trans est un produit difficile à obtenir et en outre la double liaison a tendance au cours de la condensation à s'isomériser en liaison cis. En plus, la condensation de l'éthynyl β ionol avec le chloroester en C₅ donne, d'après nos propres exemples, un dérivé acétylénique dont environ 20 % de produit de condensation est un produit secondaire branché irrécupérable de formule suivante :

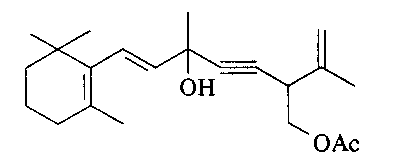

La présente invention a permis de remédier aux inconvénients de l'art antérieur. Elle permet de partir d'un dérivé halogéné en C₅ quelque soit son isomérie cis ou trans et elle ne provoque l'apparition que d'une quantité limitée d'isomère acétylénique branché irrécupérable.

La présente invention concerne un procédé de préparation d'un alcool de formule (I) : 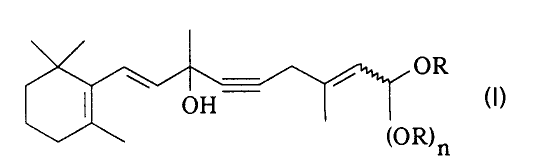 dans laquelle n est égal à 0 ou 1; lorsque n = 1, R représente un groupe allyle contenant 1 à 4 atomes de carbone ou forme avec l'autre groupe R un motif alkylidène contenant 1 à 10 atomes de carbone; lorsque n = 0, R représente une double liaison, caractérisé en ce que l'on condense l'éthynyl bétaionol avec un chloroacétal de formule (II) 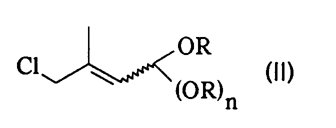 dans lequel R et n ont la même signification que dans la formule (I), en présence de cuivre.

Le procédé de préparation spécifique de la vitamine A est caractérisé en ce que dans une première étape, on condense l'éthynyl bétaionol avec le dialkoxy-1, 1 chloro-4 méthyl-3 butène-2 en présence d'un catalyseur à base de cuivre en quantité catalytique et d'une base ; dans une deuxième étape, on réalise une hydrogénation du composé obtenu à la première étape en présence de palladium supporté ; dans une troisième étape on obtient le rétinal par élimination du groupe hydroxyle et hydrolyse de la fonction acétal et dans une quatrième étape, on redresse le rétinal obtenu par complexation avec une hydroquinone.

Le schéma réactionnel de la première étape est le suivant : 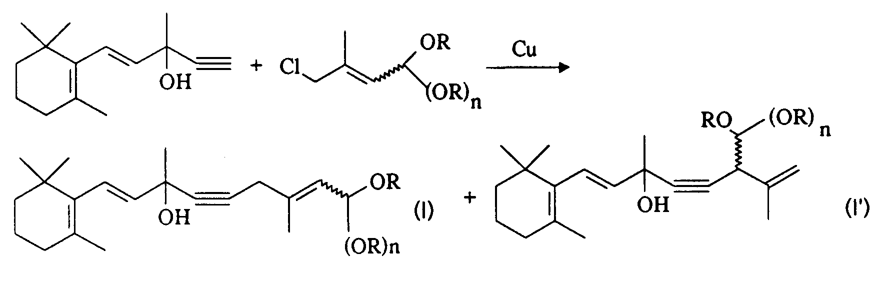 dans les formules ci-dessus n est égal à 0 ou 1 et quand n = 1 R représente un groupe alkyle contenant 1 à 4 atomes de carbone ou deux groupes R forment un groupe alkylidène contenant 1 à 10 atomes de carbone ou lorsque n = 0, R représente une double liaison.

Les composés (I) et (I') obtenus lors de cette première étape sont nouveaux.

La condensation est réalisée en présence d'un catalyseur à base de cuivre sans formation préalable du composé de condensation de l'acétylénique avec le cuivre.

Le catalyseur à base de cuivre est choisi de préférence parmi les dérivés du cuivre à l'état d'oxydation I ou II tels que les halogénures, les nitrates, les alcoolates, le cyanure, l'oxyde ou le thiocyanate de cuivre. On préfère utiliser les halogénures de cuivre tels que le chlorure, le bromure ou l'iodure cuivreux.

Pour une meilleure mise en oeuvre de l'invention on préfère utiliser un cocatalyseur à base d'une phosphine monodentate ou bidentate telle que la tributyl phosphine, la triphénylphosphine, une tritolylphosphine, une tris-p-méthoxy phénylphosphine, une diméthylaminophénylphosphine, les bisdiphénylphosphinyl 1,2 cyclobutane, 1,4 butane, le 1,3 propane et 1,2 éthane, la tricyclohexylphosphine, la tributyl phosphine, la bipyridine, l'hexaméthylphosphorotriamide, l'acétylacétate et la trisdioxaheptylamine.

La quantité de cuivre introduite pour la réaction de condensation est catalytique c'est-à-dire que le rapport nombre d'atome de cuivre au nombre de mole d'éthynyl bétaionol est compris entre 0,1 et 20 % et de préférence compris entre 0,5 et 10 %. La quantité utilisée dans l'art antérieur est largement supérieure à 1 puisque le dérivé organocuivré stoechiométrique doit être préparé préalablement à la mise en contact avec l'ester halogéné en C₅, la présente invention permet donc une économie importante dans la quantité de catalyseur utilisé et évite l'utilisation de magnésium.

Le rapport molaire entre les deux réactif mis en présence, c'est-à-dire le chloroacétal en C₅ et l'éthynyl β ionol est compris entre 1 et 5 et est de préférence compris entre 1 et 1,5.

La base utilisée est choisie de préférence parmi les acétates, les carbonates, les phosphates, les hydroxydes alcalins ou alcalinoterreux ou les amines tertiaires. On utilise 1 à 5 équivalents de base par rapport au chloroacétal.

Le composé de formule (I) obtenu après la première étape du procédé est ensuite hydrogéné selon la réaction suivante :

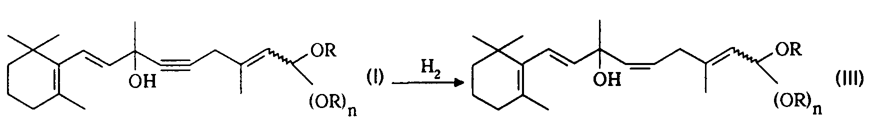

Le catalyseur d'hydrogénation est choisi parmi les catalyseurs à base de palladium ou de nickel empoisonnés encore appelés catalyseurs de Lindlar. L'empoisonnement peut être réalisé avec du plomb, une amine telle que la pyridine, la diéthylamine, la quinoléine, un sel de baryum ou de zinc. On peut aussi utiliser comme catalyseur un iodure alcalin.

On préfère utiliser un catalyseur déposé sur un support choisi parmi l'alumine, la silice ou le noir de carbone.

Le dérivé de formule (III) 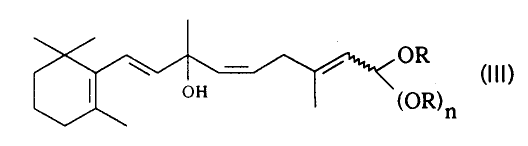 obtenu lors de cette deuxième étape est aussi un composé nouveau.

Le dérivé de formule (III) subit ensuite une élimination 1,4, et une déprotection par action catalytique d'un acide fort dans un milieu acétone/eau. On obtient alors le rétinal tout trans sur les liaisons 7-8, 11-12 mais dont la liaison 13-14 présente l'isomérie du composé C₅ de départ. Ce composé est facilement redressé par formation d'un complexe avec une hydroquinone selon la technique décrite dans le brevet FR 1.291.622.

L'utilisation de la fonction acétal permet à la fois d'accéder au rétinal et de limiter la quantité d'isomère branché (I').

La présente invention sera plus complètement décrite à l'aide des exemples suivants.

### EXEMPLE 1 Prénylation de l'éthynyl-β-ionol

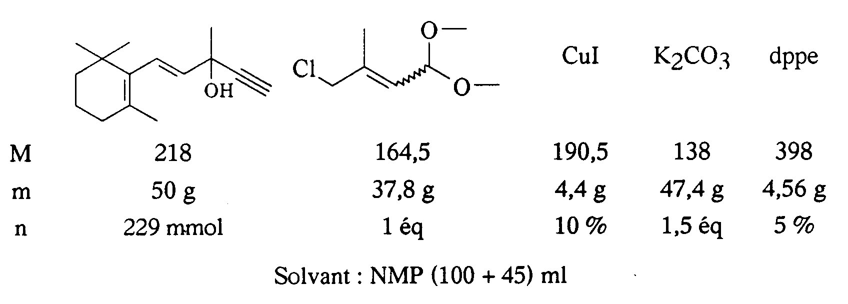

### Protocole

Sous argon, à une solution de C15 dans 100 ml de NMP, on ajoute successivement K₂CO₃ et CuI, en poudres finement broyées. L'addition de CuI provoque une exothermie de 20 à 36°C. Après 5 minutes à température ambiante, le milieu réactionnel est devenu vert. On ajoute alors rapidement une solution de chloroacétal C5 dans 45 ml de NMP puis le bisdiphénylphosphinoéthane.

L'évolution du milieu réactionnel peut être suivie en chromatographie liquide. Après 19 heures à température ambiante, le milieu réactionnel (teinte rouge) est versé sur 300 g de glace et extrait à l'éther isopropylique. La phase organique est ensuite lavée par une solution aqueuse saturée en NaCl, séchée sur sulfate de magnésium, filtrée et concentrée. On obtient 77,5 g d'une huile rougeâtre et visqueuse, composée de : 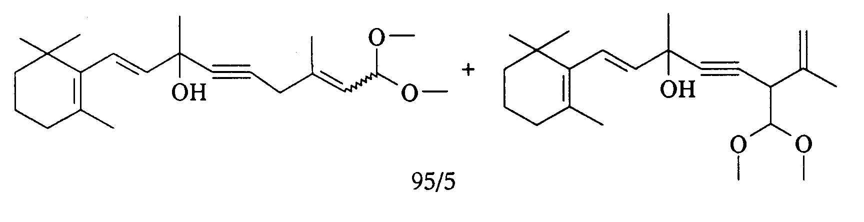 Le rendement est de 98%. Le tableau suivant résume les essais effectués en modifiant la nature du catalyseur et/ou du cocatalyseur.

**TABLEAU 1**

| Solvant | Catalyseur (%) et *cocatalyseur (%)* | Ligand (%) | Température, temps | TT (C₁₅) % | RR en C₂₀ % |
|---|---|---|---|---|---|
| NMP | CuI (10) | dppe (5) | 22°C, 19 h | ≈ 100 | 98 |
| DMF | CuI (10) | Pϕ₃ (20) | 22°C, 24 h | >80 | 66 |
| | | | 60° C, 5 h | | |
| DMF | CuI (10) | Pϕ₃ (20) | 22° C, 20 h | 76 | 68 |
| DMF | CuI (10) | - | 22°C, 24 h | < 40 | < 40 |
| NMP | CuI (10) | - | 22°C, 37 h | 93 | 69 |
| MeCN | CuI (10) | - | 22°C, 48 h | < 30 | < 30 |
| DMSO | CuI (10) | - | 22°C, 40 h | 84 | 70 |
| Sulfolane | CuI (10) | dppe (5) | 22°C, 48 h | 83 | 84 |
| DMF | CuI (10) | - | 22°C, 48 h | < 50 | < 50 |
| | Ni(Pϕ₃)₄ (5) | | 60° C, 4h30 | | |
| DMF | CuI (10) | - | 22°C, 48 h | < 50 | < 50 |
| | Pd (Pϕ₃)₄ (5) | | 60° C, 4h30 | | |
| DMF | CuI (10) | - | 22°C, 48 h | 76 | 47 |
| | RhCl (Pϕ₃)₄ (5) | | | | |
| NMP | CuCl (10) | Pϕ₃ (5) | 22°C, 40h | 43 | 14 |
| NMP | CuBrMe₂S(5) | Pϕ₃ (5) | 22°C, 40 h | | 25 |
| NMP | CuNO₃(Pϕ₃) (10) | - | 22°C, 40 h | | 25 |
| NMP | CuI (10) | Pϕ₃ (5) | 22°C, 40 h | 88 | 79 |
| DMF | CuI (10) | Pϕ₃ (5) | 22°C, 36 h | 88 | 79 |
| DMSO | CuI (10) | Pϕ₃ (5) | 22°C, 26 h | 90 | 78 |
| DMF | CuI (10) | PCy₃ (5) | 22° C, 36 h | 90 | 76 |
| DMF | CuI (10) | PBu ₃ (5) | 22°C, 36 h | 87 | 76 |
| NMP | CuI (10) | dppe (5) | 22°C, 27 h | 90 | 82 |
| NMP | CuI (10) | Bipy (5) | 26 h | 88 | 79 |
| NMP | CuI (10) | HMPA (5) | 22°C, 27 h | 76 | 44 |
| NMP | CuI (10) | Acac (10) | 22°C, 27 h | 77 | 53 |
| NMP | CuI (10) | TDA₁ (5) | 22°C, 27 h | 75 | 70 |
| NMP | CuI (20) | Pϕ₃ (5) | 22°C, 27 h | 84 | 74 |
| NMP | CuI (10) | Pϕ₃ (5) | 22°C, 40 h | 88 | 79 |
| NMP | CuI (5) | Pϕ₃ (5) | 22°C, 39 h | 88 | 63 |
| NMP | CuI (1) | Pϕ₃ (5) | 22°C, 31 h | 60 | 59 |
| NMP/H₂0 | CuI (10) | Pϕ₃ (5) | 22°C, 24 h | 81 | 55 |
| NMP | CuI (10) | PhSH (5) | 22°C, 24 h | 82 | 60 |
| dppe : bisdiphenylphosphinyl 1, 2 éthane bipy : bipyridine HMPA : hexamethylphosphorotriamide acac : acétylacétate TDA₁ : trisdioxaheptylamine | | | | | |

### EXEMPLE 2 Prénylation de l'éthynyl-β-ionol

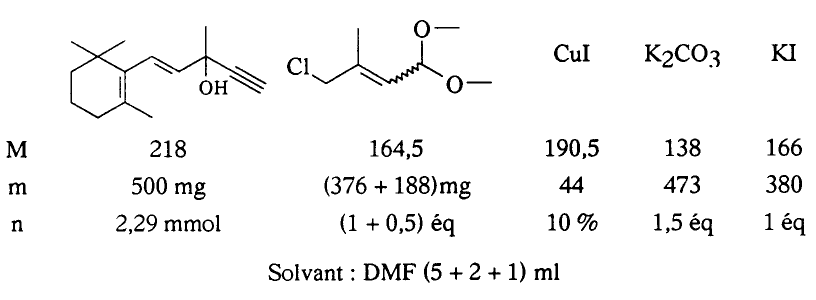

### Protocole

L'éthynyl-β-ionol est nus en solution dans 5 ml de DMF, puis on ajoute KI, K₂CO₃ et CuI sous argon.

Après 5 mn à température ambiante, on ajoute une solution de 1 équivalent molaire de C₅ dans 2 ml de DMF en un goutte à goutte très lent (environ 8 h). Après un total de 18 h à température ambiante, le C₅ a intégralement disparu. On traite alors par 5 ml d'eau puis extrait par 2 fois 8 ml d'éther, sèche sur MgSO₄ puis filtre et concentre.

Le C₂₀ attendu est purifié par chromatographie sur silice (Eluant : Pentane-Ether : 75-25). Le rendement est de 84 % en produit isolé.

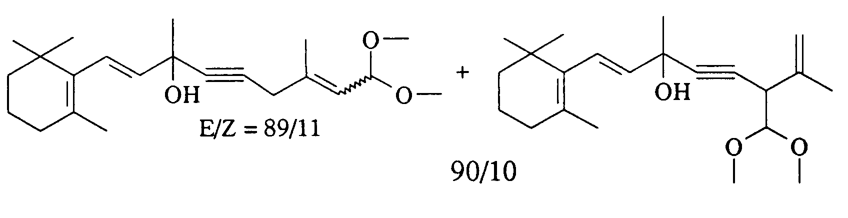

### EXEMPLE COMPARATIF Prénylation de l'éthynyl béta ionol

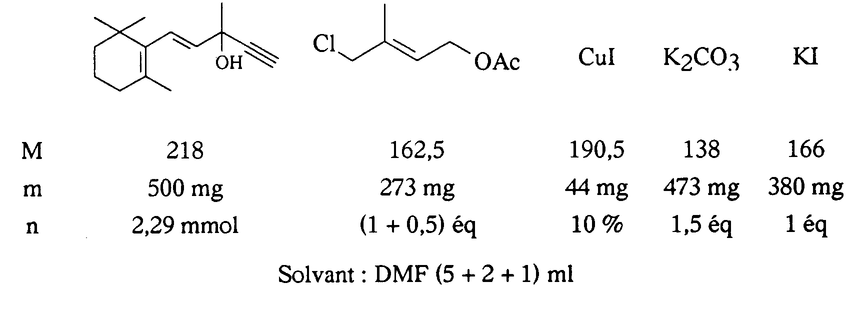

Le mode opératoire est identique à celui décrit à l'exemple 2. Le taux de transformation de l'éthynyl béta ionol est de 82%. Le rendement est de 75%.

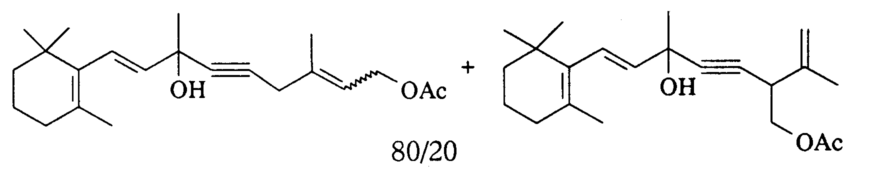

### EXEMPLE 3 Hydrogénation partielle de l'hydroxyacétal en C20

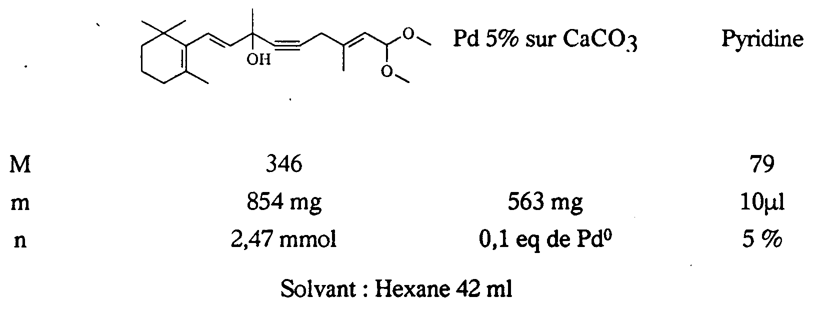

A une solution de C20 dans l'hexane obtenue à l'exemple 1, on ajoute successivement le catalyseur et la pyridine puis on place sous atmosphère d'hydrogène pendant une quinzaine d'heures. On filtre puis on concentre. Le rendement brut est de 96%.

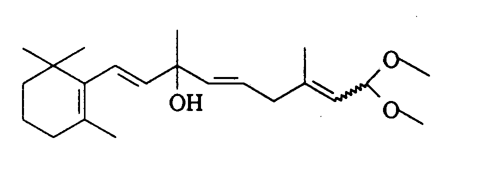

### EXEMPLE 4 Hydrolyse de l'hydroxyacétal en rétinal

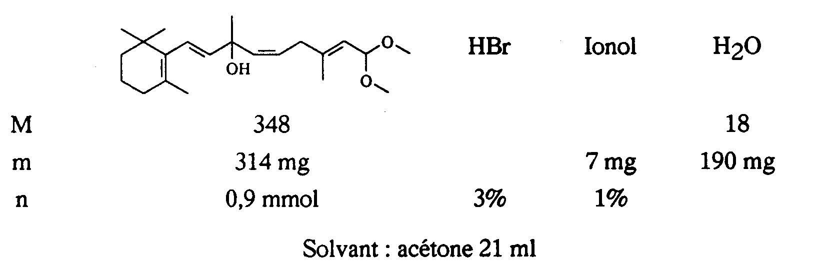

A une solution de l'hydroxyacétal en C20 et de l'ionol, on ajoute 190 µl d'eau puis on porte au reflux pendant 5 minutes. On ajoute alors rapidement 140 µl d'une solution composée de 1,5 ml d'acide bromhydrique à 48% en poids dans l'eau et 70,5 ml d'acétone.

Après 5 minutes au reflux, le C20 de départ a disparu. On verse alors sur 20 g de glace et 20 g d'eau puis on extrait à l'éther et sèche sur MgSO₄. On recueille le rétinal avec un rendement de 73% après chromatographie sur silice (Eluant : Pentane-Ether : 85-15).

## Revendications

1. Procédé de préparation d'un alcool de formule (I) : 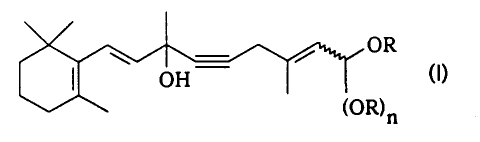 dans laquelle n est égal à 0 ou 1; lorsque n = 1, R représente un groupe alkyle ayant 1 à 4 atomes de carbone ou deux groupes R peuvent représenter un motif alkylidène contenant 1 à 10 atomes de carbone, lorsque n = 0, R représente une double liaison caractérisé en ce que l'on condense l'éthynyl β ionol avec un chloroacétal de formule (III) : 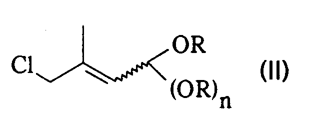 dans laquelle R et n ont la même signification que précédemment en présence de cuivre.

2. Procédé de préparation de vitamine A caractérisé en ce que dans une première étape, on condense l'éthynyl bétaionol avec le dialkoxy-1, 1 chloro-4 méthyl-3 butène-2 en présence d'un catalyseur à base de cuivre, en quantité catalytique, et d'une base ; dans une deuxième étape, on réalise une hydrogénation du composé obtenu à la première étape en présence de palladium supporté; dans une troisième étape on obtient le rétinal par élimination du groupe hydroxyle et hydrolyse de la fonction acétal et dans une quatrième étape, on redresse le rétinal obtenu par complexation avec une hydroquinone.

3. Procédé selon la revendication 2 caractérisé en ce que le catalyseur à base de cuivre est choisi parmi les halogénures, les nitrates, les alcoolates, le cyanure, l'oxyde ou le thiocyanate de cuivre à l'état d'oxydation 1 ou 2.

4. Procédé selon la revendication 3 caractérisé en que que le catalyseur à base de cuivre est choisi parmi les halogénures de cuivre et de préférence le chlorure cuivreux.

5. Procédé selon l'une quelconque des revendications précédentes caractérisé en ce qu'on utilise un cocatalyseur à base d'une phosphine.

6. Procédé selon la revendication 2 caractérisé en ce que la quantité catalytique de cuivre est comprise entre 0,1 et 20 % équivalent molaire par rapport à l'éthynyl bétaionol.

7. Procédé selon la revendication 2 caractérisé en que que la base est choisie parmi les carbonates, les acétates alcalins ou alcalinoterreux, les amines tertiaires ou les hydroxydes alcalins.

8. Procédé selon la revendication 7 caractérisé en ce que la quantité de base utilisée est comprise entre 1 et 5 équivalents molaires par rapport au chloroacétal.

9. Procédé selon la revendication 2 caractérisé en ce que la deuxième étape est réalisée en présence de palladium sur charbon et en présence de pyridine.

10. Procédé selon la revendication 2 caractérisé en ce que la troisième étape est réalisée en présence d'acide bromhydrique dans l'acétone.

11. Procédé selon la revendication 2 caractérisé en ce que la quatrième étape est réalisée en présence d'iode.

12. Intermédiaires pour préparer la vitamine A de formule (I) suivante : 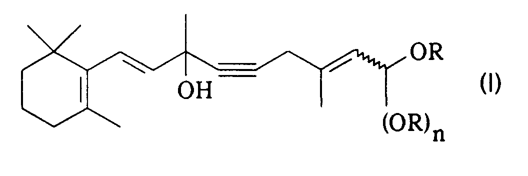 dans laquelle, lorsque n = 1, chaque groupe R représente un groupe alkyle contenant 1 à 4 atomes de carbone ou deux groupes R forment ensemble un groupe alkylidène contenant 1 à 10 atomes de carbone; lorsque n est égal à zéro R représente une double liaison.

13. Intermédiaires de formule (III) suivante : 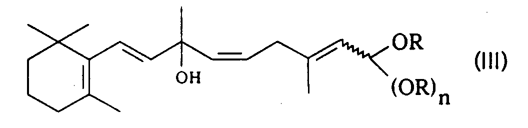 dans laquelle dans laquelle n est égal à 0 ou 1; lorsque n = 1, R représente un groupe alkyle ayant 1 à 4 atomes de carbone ou deux groupes R peuvent représenter un motif alkylidène contenant 1 à 10 atomes de carbone, lorsque n = 0, R représente une double liaison.

## Claims

1. Process for the preparation of an alcohol of formula (I) : 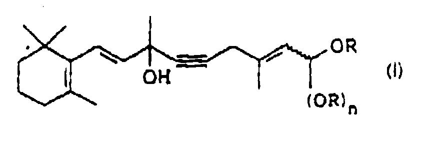 in which n is equal to 0 or 1; when n = 1, R represents an alkyl group having 1 to 4 carbon atoms or two R groups can represent an alkylidene unit containing 1 to 10 carbon atoms; when n = 0, R represents a double bond, characterized in that ethynyl-β-ionol is condensed with a chloroacetal of formula (II) : 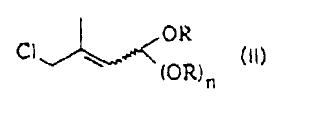 in which R and n have the same meaning as above, in the presence of copper.

2. Process for the preparation of vitamin A, characterized in that, in a first stage, ethynyl-β-ionol is condensed with the 1,1-dialkoxy-4-chloro-3-methyl-2-butane in the presence of a copper-based catalyst, in a catalytic amount, and of a base; in a second stage, the compound obtained in the first stage is hydrogenated in the presence of supported palladium; in a third stage, retinal is obtained by elimination of the hydroxyl group and hydrolysis of the acetal functional group and, in a fourth stage, the retinal obtained is rectified by complexing with a hydroquinone.

3. Process according to claim 2, characterized in that the copper-based catalyst is chosen from copper halides, nitrates, alkoxides, cyanide, oxide or thiocyanate, the copper being in the 1 or 2 oxidation state.

4. Process according to claim 3, characterized in that the copper-based catalyst is chosen from copper halides and preferably cuprous chloride.

5. Process according to any one of the preceding claims, characterized in that use is made of a cocatalyst based on a phosphine.

6. Process according to claim 2, characterized in that the catalytic amount of copper is between 0.1 and 20 molar equivalent % with respect to ethynyl-β-ionol.

7. Process according to claim 2, characterized in that the base is chosen from alkali metal or alkaline-earth metal carbonates or acetates, tertiary amines or alkali metal hydroxides.

8. Process according to claim 7, characterized in that the amount of base used is between 1 and 5 molar equivalents with respect to the chloroacetal.

9. Process according to claim 2, characterized in that the second stage is carried out in the presence of palladium-on-charcoal and in the presence of pyridine.

10. Process according to claim 2, characterized in that the third stage is carried out in the presence of hydrobromic acid in acetone.

11. Process according to claim 2, characterized in that the fourth stage is carried out in the presence of iodine.

12. Intermediates for preparing vitamin A of following formula (I) : 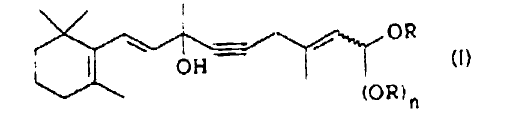 in which, when n = 1, each R group represents an alkyl group containing 1 to 4 carbon atoms or two R groups together form an alkylidene group containing 1 to 10 carbon atoms; when n is equal to 0, R represents a double bond.

13. Intermediates of following formula (III) : 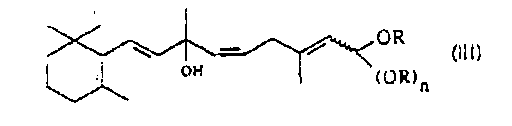 in which n is equal to 0 or 1; when n = 1, R represents an alkyl group having 1 to 4 carbon atoms or two R groups can represent an alkylidene unit containing 1 to 10 carbon atoms; when n = 0, R represents a double bond.

## Patentansprüche

1. Verfahren zur Herstellung eines Alkohols der Formel (I): 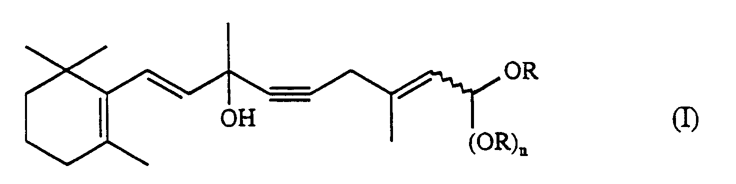 in der n gleich 0 oder 1 ist; falls n = 1 ist, so stellt R eine Alkylgruppe mit 1 bis 4 Kohlenstoffatomen dar, oder es können zwei R-Gruppen eine Alkylideneinheit mit 1 bis 10 Kohlenstoffatomen bilden, falls n = 0 ist, so stellt R eine Doppelbindung dar,
dadurch gekennzeichnet, daß Ethinyl-β-Ionol in Gegenwart von Kupfer mit einem Chloracetal der Formel (II) kondensiert wird: 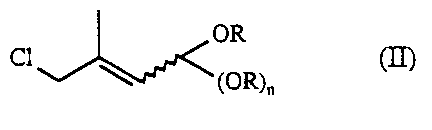 in der R und n dieselbe Bedeutung haben wie oben.

2. Verfahren zur Herstellung von Vitamin A, dadurch gekennzeichnet, daß in einem ersten Reaktionsschritt Ethinyl-β-Ionol in Gegenwart einer katalytischen Menge eines Katalysators auf Kupferbasis und einer Base mit 1,1-Dialkoxy-4-chlor-3-methyl-buten-(2) kondensiert wird; in einem zweiten Reaktionsschritt wird in Gegenwart von auf einem Träger fixierten Palladium eine Hydrierung der im ersten Reaktionsschritt erhaltenen Verbindung durchgeführt; in einem dritten Reaktionsschritt erhält man durch Eliminierung der Hydroxylgruppe und Hydrolyse der Acetalfunktion das Retinal, und in einem vierten Reaktionsschritt wird das erhaltene Retinal durch Komplexierung mit einem Hydrochinon zur all-trans-Verbindung isomerisiert.

3. Verfahren nach Anspruch 2, dadurch gekennzeichnet, daß der Katalysator auf Kupferbasis aus den Kupferhalogeniden, -nitraten, -alkoholaten, -cyaniden, -oxiden und -thiocyanaten der Oxidationsstufen 1 und 2 ausgewählt wird.

4. Verfahren nach Anspruch 3, dadurch gekennzeichnet, daß der Katalysator auf Kupferbasis aus den Kupferhalogeniden ausgewählt wird und vorzugsweise Kupfer(I)-chlorid ist.

5. Verfahren nach einem der vorhergehenden Ansprüche, dadurch gekennzeichnet, daß ein Cokatalysator auf der Basis eines Phosphins verwendet wird.

6. Verfahren nach Anspruch 2, dadurch gekennzeichnet, daß die katalytische Menge an Kupfer zwischen 0,1 und 20 mol-%, bezogen auf das Ethinyl-β-ionol, liegt.

7. Verfahren nach Anspruch 2, dadurch gekennzeichnet, daß die Base aus den Alkali- und Erdalkalicarbonaten und -acetaten, den tertiären Aminen und den Alkalihydroxiden ausgewählt wird.

8. Verfahren nach Anspruch 7, dadurch gekennzeichnet, daß die verwendete Basenmenge zwischen 1 und 5 mol pro Mol Chloracetal liegt.

9. Verfahren nach Anspruch 2, dadurch gekennzeichnet, daß der zweite Reaktionsschritt in Gegenwart von Palladium auf Aktivkohle und in Gegenwart von Pyridin durchgeführt wird.

10. Verfahren nach Anspruch 2, dadurch gekennzeichnet, daß der dritte Reaktionsschritt in Gegenwart von Bromwasserstoffsäure in Aceton durchgeführt wird.

11. Verfahren nach Anspruch 2, dadurch gekennzeichnet, daß der vierte Reaktionsschritt in Gegenwart von Iod durchgeführt wird.

12. Zwischenprodukte der Herstellung von Vitamin A der folgenden Formel (I): 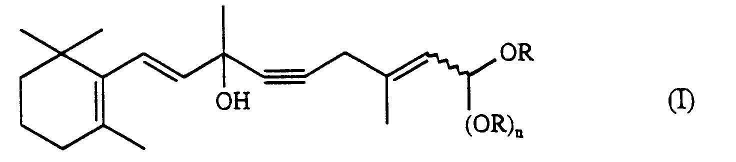 in der, falls n = 1 ist, jede R-Gruppe eine Alkylgruppe mit 1 bis 4 Kohlenstoffatomen darstellt oder zwei R-Gruppen zusammen eine Alkylidengruppe mit 1 bis 10 Kohlenstoffatomen bilden, oder, falls n = 0 ist, R eine Doppelbindung darstellt.

13. Zwischenprodukte der folgenden Formel (III): 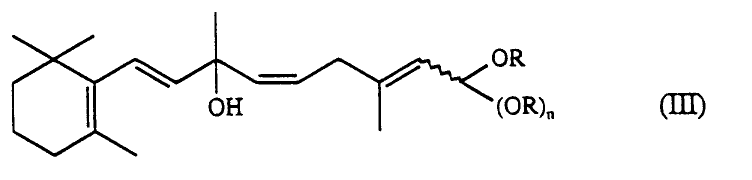 in der n gleich 0 oder 1 ist; falls n = 1 ist, so stellt R eine Alkylgruppe mit 1 bis 4 Kohlenstoffatomen dar, oder es können zwei R-Gruppen eine Alkylideneinheit mit 1 bis 10 Kohlenstoffatomen bilden, falls n = 0 ist, so stellt R eine Doppelbindung dar.
